# EUROPEAN PATENT APPLICATION

(11) **EP 2 281 870 A1**
(43) Date of publication of application: **09.02.2011**
(21) Application number: 10183968.6
(22) Date of filing: 17.03.2001
(51) Int. Cl.: C12N 1/20, C12N 1/16, C12M 1/00, A23C 9/123, A23C 19/032

(54) **Method for supply of starter cultures having a consistent quality**

(30) Priority: 21.03.2000 DK 200000474; 21.03.2000 US 191307 P
(62) Divisional of application: 01916929.1
(71) Applicant: Chr. Hansen A/S, 2970 Hoersholm (DK)
(72) Inventor: Kringelum, Børge, 2750 Ballerup (DK); Kringel, Maibritt, 91740 Chalou-Moulineux (FR); Nielsen, Knus Striib, 2650 Hvidovre (DK)
(74) Representative: Hagen, Klaus Bach

(57) **Abstract**

The present invention relates to the field of producing starter cultures. In particular, a method for customers in need of a starter culture with a consistent quality, is provided. Specifically, the method involves the use of subsets of a stock inoculum material, which comprises a concentrate of starter culture organism cells to be propagated for direct inoculation of a cultivation medium, to obtain a starter culture whereby the conventional stepwise preparation of inoculum material for the production of a starter culture can be avoided. This novel method can be used for the manufacturing of starter cultures for the food, feed or pharmaceutical industry. Furthermore, the method is useful in the cultivation of cells expressing desired products, such as primary and secondary metabolites, including e.g. enzymes and flavours.

## Description

### FIELD OF INVENTION

The present invention relates to the field of producing starter cultures. In particular, a method for production of starter cultures with a consistent quality has been developed for the food, feed or pharmaceutical industry. Specifically, the method involves the use of subsets of a stock inoculum material which comprises a concentrate of cells to be propagated for direct inoculation of a cultivation medium to obtain a starter culture whereby the conventional and less profitable stepwise preparation of inoculum material for the production of a starter culture can be avoided.

### TECHNICAL BACKGROUND OF THE INVENTION

Microbial cultures are used extensively for fermentations in the industry, both in the manufacturing of food, feed and pharmaceutical products, and in the manufacturing of specific products, such as enzymes, primary and secondary metabolites.

Although the majority of fermentation processes still relies on inocula naturally occurring in the fermentation medium, most fermentations are now based on the use of dried, frozen or freeze-dried microbial inoculation media. Inoculation material is produced in small ampoules and distributed to the fermentation plants and each plant often makes several steps to be able to inoculate large fermenters in which the product is produced by fermentation.

In the conventional production of starter cultures, the cultivation of cells involves an inoculation procedure where the final cultivation medium is inoculated with an appropriate number of the cells (the inoculum material) to be propagated.

According to presently used working procedures the inoculum material is prepared using a stepwise or successive propagation starting from a generally small amount of inoculum material, also referred to as a mother culture or a primary inoculum material. This inoculation procedure typically involves 2 to 4 propagation steps, using increasing volumes of medium in order to obtain sufficient inoculum material for the inoculation of the final cultivation medium for the starter culture organism.

However, the fermentation industry, which currently uses this procedure of producing inoculum materials, is confronted with several problems. One significant problem is that each step in the procedure leading to the production of the final inoculum material, i.e. the transfer of the inoculum from one volume to a relatively larger volume, involves a serious risk of contamination of the inoculum material with undesired organisms such as organisms from other fermentations, i.e. cross contamination, and spoilage bacteria, e.g. *Bacil*lus species or Gram-negative bacteria, or bacteriophages.

In addition to the risk of contaminating the inoculum material, the use of the above described procedure involves consideration of how to diminish or circumvent the following problems and/or disadvantages: (i) the preparation of the inoculum material is very labour intensive, and in addition occupies relatively much space and equipment, (ii) the propagation of the mother culture encompasses several intermediate steps to obtain the final inoculum material and it takes at least 36 hours which necessitates a high degree of production planning, i.e. a tight and inflexible working schedule is mandatory, (iii) the working procedure adhered to implies a high degree of manual handling, and (iv) the stepwise propagation has to be performed in a regular and frequent manner, which leaves no time for subjecting the inoculum material to various quality tests prior to its use. Thus, the inoculum material may easily be contaminated or contain starter culture organism different to the one contemplated.

Hence, by making each starter culture production from a mother culture there is a risk of a high variation between the quality of separately produced batches of the final inoculum material , both in-house and between factories and various plants within or outside the producing company, i.e. high variation with regard to quality of the fermentation end products made by use of commercial starter cultures.

In the fermentation industry, there is a clear trend towards a high flexibility in the production planning, high quality of the end products and a high reproducibility between the individual production batches of starter cultures. Furthermore, there is an exhorbitant demand for improved production methods which reduce manpower and time, and thus expenses.

Therefore, there is a clear need for an improved procedure for inoculating a final cultivation medium which is not only adapted to the increasing demand for strict control of contamination during production of commercial starter cultures or desired products, and a high consistency of the quality hereof, but which also implies that the above problems associated with the currently used method of preparing inoculum materials can be reduced or eliminated. The method which is provided herein implies a high degree of flexibility, and the production time and manpower are reduced considerably. Furthermore, the problem associated with batch to batch variation is decreased, as the inoculation system as provided herein permits central preparation of large batches of stock inoculum materials which, if required, can be stored for extended periods of time.

### SUMMARY OF THE INVENTION

It is the primary objective of the present invention to provide, a one-step, direct inoculation procedure which meets the demand from the fermentation industry of higher flexibility, better quality management of the end products, and a higher reproducibility between production batches.

The present invention provides a method for supply to customers in need of a starter culture characterised by a consistent quality. The method disclosed in the present invention comprises the following steps :
(i) supply of a stock inoculum material comprising a concentrate of starter culture organism cells;
(ii) use of, for subsequent production of starter cultures, a subset of said stock inoculum material for direct inoculation of a cultivation medium for said starter culture organism.
(iii) propagation of the cells of the starter culture organism for a period of time adjusted sufficiently in size to produce a desired amount of said cells; and
(iv) harvest of the propagated cells to provide a starter culture.

### DETAILED DISCLOSURE OF THE INVENTION

Thus, in its broadest aspect the invention provides a method for supply to customers in need of a starter culture with a consistent quality. The expression "customers in need of a starter culture" relates to the food, feed or pharmaceutical industry which are the major producers, vendors and purchers of microbial fermented products. Their desire is to provide high quality products to their customers. Such products could be starter cultures, enzymes, pharmaceuticals, vitamins, and amino acids, and are in general used for the production of specific products, e.g. in the food industry for the production of fermented food products including milk products such as cheese and butter, as they impart desired organoleptic and sensory and other quality features to said products by performing a number of different functions.

As mentioned above, it is an important objective of the present invention to provide a method for starter cultures wherein the variation between the quality of separately produced batches of the same starter culture organisms is reduced, i.e. to provide starter cultures with a consistent performance and a high reproducibility in terms of quality. The expression "starter cultures having a consistent quality" relates to starter cultures, which, when produced from the same stock inoculation material, and regardless of when or where they are produced, substantially will have the same uniform performance quality, i.e. having substantially the same metabolic activity and containing substantially the same number of cells per ml and composition hereof.

The first step of the method according to the invention is to provide a stock inoculum material comprising a concentrate of the chosen production strain or strains. In the present context, the expression "stock inoculum material" refers to a quantity of inoculum material which after dividing it into subsets can be stored, and thus be immediately available for use as direct inoculum material for the production of a starter culture. In practice, small portions, i.e. subsets of the stock inoculum material, are used individually for direct inoculation of a cultivation medium. Because it is possible to store the stock inoculum material, a given propagation factory can produce the stock inoculum material some time before use, and thus possess sufficient time to subject the inoculum to various quality tests. By having a stock or a supply of the same inoculum material available, the propagation factory is able to produce starter cultures at any time and of a high and consistent quality, because the starting material for the different starter culture productions, i.e. the subsets of the stock inoculum material, originates from the same stock and thus is substantially always the same. Furthermore, as the subset can be used for direct inoculation of the cultivation material there is no risk of contamination of the production strain or strains used as the starter culture, and the propagation plant can be certain that the chosen starter culture is actually propagated in the cultivation medium. In a preferred embodiment, the stock inoculum material comprises a substantial number of subsets which are individually sealed and enclosed as disclosed below.

According to the present invention, the stock inoculum material can be stored at appropriate conditions for at least 24 hours, such as at least 36 hours, e.g. at least 48 hours including at least 72 hours prior to being added to the cultivation medium, while substantially maintaining the viability and/or metabolic activity of the cells. However, it may be desirable to store the inoculum material for at least 1 month, such as for at least 2 months, e.g. for at least 4 months, including for at least 5 months, such as for at least 6 months. As mentioned in the Examples below, the inoculum material may even be stored for at least 1 year, such as at least 2 years, e.g. at least 4 years including 5 years, such as at least 6 years. As used herein, the expression "substantially maintaining the viability and/or metabolic activity" implies that at least 50% of the initial viability and/or metabolic activity is maintained, such as at least 60%, 70%, 80% or 90% hereof.

It is important to note that it is possible to store the stock inoculum material in a liquid state for the above period of time while maintaining its initial activity during storage. However, this may require the incorporation of an effective amount of a metabolic activity stabilising compound.

It is to be understood that the size of the stock inoculum material depends on the type of inoculum material and the customer demand for a particular starter culture. However, in useful embodiments, the stock inoculum material provided in step (i) of the present method is in quantities sufficient to inoculate at least 20 kg of cultivation medium, such as at least 50 kg of cultivation medium, such as at least 100 kg of cultivation medium, e.g. at least 200 kg of cultivation medium including at least 300 kg of cultivation medium, such as at least 500 kg of cultivation medium, such as at least 1000 kg of cultivation medium, e.g. at least 5,000 kg of cultivation medium including at least 10,000 kg of cultivation medium, such as at least 20,000 kg of cultivation medium, such as at least 30,000 kg of cultivation medium, e.g. at least 80,000 kg of cultivation medium including at least 180,000 kg of cultivation medium.

However, in further useful embodiments, the stock inoculum material provided in step (i) of the present invention is in quantities sufficient to supply all starter culture propagation plants for at least 3 months with starter cultures havintg a consistent quality, such as at least 5 months, e.g. at least 8 months, including at least 1 year, such as at least 2 years, e.g. at least 5 years, including at least 10 years.

For the purpose of the description of the present invention, the expression "direct inoculation" indicates that the inoculum material used for inoculating the cultivation medium is not, as it is currently used, provided by a series of successive steps of propagation of primary inoculum material, but is provided in appropriate portions of the stock inoculum material which contain sufficient amounts, i.e. in a sufficient concentration of viable cells, to directly inoculate a fermenter with cultivation medium for the production of a starter culture. This implies that the total period of time for producing a starter culture is considerably reduced.

The stock inoculum material and/or the subset thereof may conveniently be in a liquid, semi-liquid, frozen or dried state, such as e.g. freeze-dried or spray-dried. It will be understood that if the inoculum material is frozen, the material may be thawed prior to being added to the cultivation medium in step (ii) of the present method. The thawing may be conducted by e.g. using a water bath or a microwave apparatus. Furthermore, the inoculum material may be combined with an aqueous medium to obtain a suspension of the cells before adding it to the cultivation medium in step (ii). The aqueous medium may be water including tap water, distilled water or deionized water, or it can be any aqueous medium which is suitable for suspending a cell culture such as suspensions of milk solids, whey or solutions containing organic compounds and/or derivatives such as different salts. The aqueous medium can further comprise buffering agents and/or microbial nutrients. An example of a production of a stock inoculum material using the present method is described in detail below.

For subsequent production of starter cultures, the second step of the method according to the invention is the use of a subset of said stock inoculum material for direct inoculation of a cultivation medium for said production strain or strains. The expression "for subsequent production of starter cultures" indicates that by providing a relatively large quantity of inoculum material it is possible, by using portions of the stock inoculum material, i.e. subsets, to produce starter cultures which all originate from the same stock, but which may be produced at different points in time and at different locations.

The third step of the present method is the propagation of the cells of the production strain or strains for a period of time adjusted sufficiently in size to produce a desired amount of said cells. It is generally desirable that a particular period of time is selected which is appropriate for the specific starter culture microbial cells to be propagated in order to obtain the amount of cells needed for a commercial starter culture. However, in useful embodiments, such a period of time is at least 2 hours, such as at least 4 hours, e.g. at least 6 hours, including at least 10 hours, such as at least 12 hours, such as at least 24 hours including at least 36 hours. In further useful embodiments, such time period is in the range of 2 to 36 hours, such as in the range of 4 to 24 hours, e.g. in the range of 6 to 24 hours, including in the range of 10 to 24 hours or in the range of 10 to 12 hours in addition to several days for e.g. fungi. In the present context, the term "propagation" is used interchangeably with the terms "cultivation" and "fermentation" and refers to the broadest sense of these terms with respect to processes whereby biomasses of production strains are obtained. The term "production strain" refers, in the present context, to cells of any microbial species that can be used in industrial productions of starter cultures including species of bacteria including lactic acid bacteria, fungi and yeast.

It will easily be understood that the inoculation into the cultivation medium with the stock inoculum material subsets comprising a concentrate of a production strain or strains results in a propagation of the cells, and thus to the production of a product to be commercialised. For the purpose of the description of the present invention, the term "to the production of a product to be commercialised" is used in a general sense and comprises the total amount of a given cell culture that is actively growing and forming a population inhabiting a given container. In addition, the definition also encompasses the cell material produced by growth of a microorganism producing a desired product in an industrial process, such as a fermentation process, as discussed below.

Following the propagation of the production strain or strains for an appropriate period of time, the propagated cells are harvested in order to provide such cells as a starter culture to the customer in need of a starter culture for his particular production. However, it may be desirable to separate the cells from the remaining cultivation material. Thus, in a preferred embodiment of the invention, the method comprises the further step of at least partially separating the propagated cells or biomass. However, it may also be desirable to leave the starter culture in the fermenter for further production.

It will be appreciated that the cultivation medium used in step (ii) of the method according to the invention may be any conventional medium used for the propagation of microbial cells. Such a cultivation medium may be in liquid form , semi-liquid or solid medium and may comprise one or more single milk components including skimmed milk.

As mentioned above, the stock inoculum material comprises a concentrate of a production strain or strains to be used as starter culture organism cells. In the present context, the expression "a concentrate" relates to a suspension of cells or medium comprising the cells, said suspension or medium having a content of viable cells (colony forming units, CFUs) which is at least 10⁸ CFU per g, such as at least 5 x 10⁸ CFU per g, e.g. at least 10⁹ CFU per g, including at least 5 x 10⁹ CFU per g, such as at least 10¹⁰ CFU per g including at least 5 x 10¹⁰ CFU per g, e.g. at least 10¹¹ CFU per g, such as at least 5 x 10¹¹ CFU per g including at least 10¹² CFU per g, e.g. at least 5 x 10¹² CFU per g. However, in useful embodiments the concentrate has a content of viable cells which is in the range of 10⁸ to 5 x 10¹² CFU per g, such as in the range of 10⁹ to 10¹² CFU per g, e.g. in the range of 10⁹ to 5 x 10¹¹ CFU per g, including in the range of 10⁹ to 10¹¹ CFU per g, such as in the range of 10⁹ to 5 x10¹⁰ CFU per g, e.g. in the range of 10⁹ x 10¹⁰ CFU per g.

Thus, according to the present invention the inoculation, a subset of the stock inoculum material in step (ii) of the present method is inoculated directly into the cultivation medium. In advantageous embodiments, the subset of the stock inoculum material is directly inoculated in step (ii) into the cultivation medium at a rate of a maximum of 0.1 %, such as at the most 0.08%, e.g. at the most 0.05%, including at the most 0.01 %, such as at the most 0.005%, including at the most 0.001 %.

In accordance with the method of the invention, the amount of the subset of the stock inoculum material for direct inoculaton of the cultivation medium in step (ii) of the present method provides a ratio between the CFU per g of cultivation medium immediately after inoculation, and the CFU per g of the subset of the stock inoculum material being inoculated, in the range of 1:100 to 1:100,000. In the calculation of the above ratio, it is important that the calculation is based on CFU per g of cultivation medium immediately afterthe inoculation of the cells. This is achieved by collecting a sample of the inoculated cultivation medium within a short period of time, preferably within 5 minutes and subjecting the collected sample to a CFU determination using optimum conditions for this. In certain preferred embodiments, the ratio between the CFU per g of cultivation medium immediately after inoculation and the CFU per g of the inoculum material being inoculated is in the range 1:1,000 to 1:75,000, such as in the range of 1:5,000 to 1:50,000, such as in the range of 1:10,000 to 1:20,000, including in the range of 1:15,000 to 1:100,000.

In order to achieve a reasonably rapid cell propagation during cultivation, it is generally preferred to add an amount of the inoculum material to the cultivation medium that provides a number of CFUs which is at least 10⁵ CFUs per g of cultivation medium immediately after inoculation, such as at least 10⁶ CFUs per g, such as at least 10⁷ CFUs per g, e.g. at least 10⁸ CFUs per g including at least 10⁹ CFUs per g. However, in useful embodiments, the added amount of the inoculum material to the cultivation medium provides a number of CFUs that is in the range of 10⁵ to 10⁹ CFUs per g of cultivation medium immediately after inoculation, such as in the range of 10⁶ to 10⁸ CFUs per g, such as in the range of 10⁶ to 10⁷ CFUs per g.

It is important that the inoculum material is added under conditions where the material is not contaminated with other micro-organisms. Accordingly, in a preferred embodiment the inoculum material is added under substantially aseptical conditions to the cultivation medium. Means for the aseptical transfer and/or inoculation of inoculum are discussed below.

In one useful embodiment, the inoculum material is provided in a sealed enclosure, preferably non-pyrogenic, which can be made of a rigid, non-flexible or flexible material, e.g. selected from the group consisting of a polyolefin, a substituted olefin, a copolymer of ethylene, a polyester, a polycarbonate, a polyamide, a polypropylen, a polyethylene, an acrylonitrile and a cellulose derivative. The use of a flexible material implies that the packaging after loading with the concentrate can be evacuated prior to an airtight sealing to achieve a low volume . Thus, the enclosure can be provided with a sealing mechanism which can be made of a flexible material. Optionally, the enclosure can be made of a solid material e.g. selected from the group consisting of a polymers, a glass or a metal. The enclosure may also be filled with a non-atmospheric gas prior to sealing. It will be understood, that the expression "non-atmospheric gas" relates to an inert gas or to a modified atmosphere such as e.g. N₂ and CO₂. In a further useful embodiment, the sealed enclosure is made of a flexible material comprising metal foil.

The size of the packaging enclosure will i.a. depend on the production scale of the starter culture. As explained in the following, a highly advantageous feature of the invention is that the size of the enclosure can be adapted to comply with the particular needs of individual production plants. This applies both to the amount and composition of the concentrate and to the cubic content of the enclosure. Thus, in a specific embodiment, the sealed enclosure has a cubic content of at least 0.005 litres, such as at least 0.01 litres, such as at least 0.1 litres, e.g. at least 0.5 litres, such as at least 1.0 litres, e.g. at least 1.5 litres, such as at least 2 litres, e.g. at least 5 litres including at least 10 litres, e.g. at least 15 litres or at least 20 litres.

As discussed above, it is desirable to transfer the inoculum material directly to the cultivation material under aseptic conditions. Thus, in another embodiment the sealed enclosure is provided with outlet means for connecting the enclosure to the inlet means of the container comprising the cultivation medium. These outlet means permit the concentrate of cells to be substantially aseptically introduced into the container. Such outlet means may be in the form of a pipeline provided with e.g. a clean-click system or a threaded outlet, which makes it possible to connect the enclosure to the cultivation container, provided that the container is provided with inlet means which permits said connection of the enclosure. Furthermore, outlet means of the enclosure may be in a form of a tubing which may be provided with a screw for connection to the container.

In accordance with the invention, cells of any micro-organism which is of use in the industry as a starter culture can be used. Thus, in preferred embodiments, the starter culture organism in step (i) of the present method is of a species selected from the group consisting of a lactic acid bacterial species, a *Bifidobacterium* species, a *Propionibacterium* species, a *Staphylococcus* species, a *Micrococcus* species, a *Bacillus* species, an *Enterobacteriaceae* species including *Escherichia coli,* an *Actinomycetes* species, a *Corynebacterium* species, a *Brevibacterium* species, a *Pediococcus* species, a *Pseudomonas* species, a *Sphingomonas* species, a *Mycobacterium* species, a *Rhodococcus* species, a fungal species and a yeast species.

In a useful embodiment, the lactic acid bacterial species is selected from the group consisting of *Lactococcus* spp. such as *Lactococcus lactis, Lactococcus lactis* subsp. *lactis, Lactococcus lactis* subsp. *cremoris* and *Lactococcus lactis* subsp. *lactis* biovar *diacetylactis, Lactobacillus* spp. such as *Lactobacillus casei, Lactobacillus paracasei* subsp. *paracasei, Lactobacillus delbrueckii* subsp. *lactis, Lactobacillus helveticus, Lactobacillus delbrueckii* subsp. *bulgaricus* and *Lactobacillus acidophilus, Leuconostoc* spp. such as *Leuconostoc lactis, Leuconostoc mesenteroides* subsp. *mesenteroides* and *Leuconostoc mesenteroides* subsp. *cremoris, Pediococcus* spp., *Oenococcus* spp., *Enterococcus* spp. such as *Enterococcus durans* and *Enterococcus faecium,* and *Streptococcus* spp. such as *Streptococcus thermophilus.*

Also the strict anaerobic bacteria belonging to the genus *Bifidobacterium* including *Bifidobacterium bifidum, Bifidobacterium lactis* and *Bifidobacterium longum* are commonly used as strains in dairy starter cultures and are generally included in the group of lactic acid bacteria. Additionally, species of *Propionibacterium, Corynebacterium* and *Brevibacterium* are used as starter cultures, in particular in the manufacture of enzymes, pharmaceuticals, amino acids, vitamins, cheese and meat.

A further group of lactic acid bacterial species which are used as so-called probiotics include e.g. *Lactobacillus johnsonii, Lactobacillus crispatus, Lactobacillus gasseri, Lactobacillus casei, Lactobacillus paracasei* subsp. *paracasei, Lactobacillus rhamnosus, Lactobacillus reuteri, Lactobacillus plantarum Bifidobacterium infantis, Bifidobacterium adolescentis, Bifidobacterium longum, Bifidobacterium animalis, Bifidobacterium breve, Enterococcus faecium* and *Streptococcus salivarius.*

Another group of microbial starter cultures are fungal cultures, including yeast cultures and cultures of filamentous fungi, which are particularly used in the manufacture of certain types of enzumes, pharmaceuticals, amino acids, vitamins, cheese, meat and beer. Examples of currently used cultures of fungi include e.g. *Debaryomyces* species such as *Debaryomyces hansenii, Penicillium* species such as *Pencillium roqueforti* and *Penicillium candidum, Geotrichum candidum, Torula kefir, Cryphonecdria parasitica Candida valida, Kluyveromyces* species such as *Kluyveromyces maxianus* and *Kluyveromyces thermotolerans, Aspergillus* species such as *Aspergillus niger, Torelospora* species such as *Torelospora delbrueckii, Saccaromyces* species such as *Saccaromyces cerevisiae, Saccaromyces carlbergensis and Saccaromyces kefir,* Ogtsea species, *Trametes* species, Mucor species *and Rhizomucor* species, *Humicola, insolent, tricoderma etc....*

It will be appreciated that the micro-organism or the production strain can be selected from a genetically modified strain of one of the above mentioned strains or any other strain useful in the industry. As used herein, the expression "genetically modified bacterium" is used in its conventional meaning of that term, i.e. it includes strains obtained by subjecting a strain to any conventionally used mutagenization treatment including treatment with a chemical mutagen such as ethanemethane sulphonate (EMS) or N-methyl-N'-nitro-N-nitroguanidine (NTG), UV light or to spontaneously occurring mutants, including classical mutagenesis. Furthermore it is possible to provide the genetically modified organism by random mutagenesis or by selection of spontaneously occurring mutants, i.e. without the use of recombinant DNA-technology, it is envisaged that mutants of micro-organisms can be provided by such technology including site-directed mutagenesis and PCR techniques and other *in vitro* or *in vivo* modifications of specific DNA sequences once such sequences have been identified and isolated.

As often in several fermentation processes in the industry, especially the dairy industry, the biomass to be produced may comprise at least two starter culture strains, e.g. a mixture of strains of different kinds of species, such as e.g. a mixture of *Streptococcus thermophilus* and *Lactobacillus delbrueckii* subsp. *bulgaricus.*

Subsequent to the production of the propagated cells in step (iv) of the method according to the invention, the cells may be recovered from the production container and packed in order to be shipped to the fermentation industry as a commercial starter culture. Thus, in a preferred embodiment, the starter culture may be used for the inoculation of milk which is further processed to obtain a dairy product which is selected from the group consisting of cheese, yoghurt, butter, inoculated sweet milk and a liquid fermented milk product such as e.g. buttermilk or drinking yoghurt. Such further processing steps are carried out using conventional process steps. Another significant application of the starter cultures is as so-called probiotics. In the present context , the term "probiotic" is to be understood as microbial culture which, when ingested in the form of viable cells by humans or animals, confers an improved health condition, e.g. by suppressing harmful micro-organisms in the gastrointestinal tract, by enhancing the immune system or by contributing to the digestion of nutrients. A typical example of such a probiotically active product is "sweet acidophilus milk".

In an interesting embodiment, the cells being propagated in the cultivation medium express a desired gene product or produce a desired product. In the present context, the expression "desired gene product" relates to gene products and primary and/or secondary products of the cell metabolism. Such desired products include enzymes such as carbohydrases, cellulases, glycolases, pectinases, amylases, lipases, lysozymes, chymosin or proteases, and enzymes for industrial processes that include detergent, starch, food, feed, or textile enzymes. Furthermore, the above expression encompasses pharmaceutically active substances such as e.g. a hormone, insulin, an antigen, a vaccine and an interleucin. Furthermore, desired products are bacteriocins, pigments, vitamins, amino acids, emulsifiers, and flavouring compounds such as diacetyl and acetoin.

In a useful embodiment, the desired product is selected from the group consisting of a pigment and a flavouring compound, including diacetyl and acetoin, an emulsifier, a vitamin, a growth-stimulating compound, a food additive and a feed additive.

The invention will now be described in further details in the following non-limiting examples.

### EXAMPLE 1

Evaluation of the deviation of the quality of commercial starter cultures produced when using a subset of the a stock inoculum material and when using starter cultures produced by a conventional method

### 1.1 Introduction

This example shows a comparison of the deviation of the quality of commercial starter cultures when produced by using a subset of the same stock inoculum material, and when produced by a conventional method for producing a commercial starter culture, i.e. by a stepwise or successive propagation starting from a generally small amount of stock inoculum material (mother culture), and which involves 2 to 4 propagation steps using increasing volumes of cultivation medium in order to obtain a sufficient amount of inoculum material to inoculate the final cultivation medium for the production of a commercial starter culture.

### 1.2 Material and Methods

### 1.2.1 Production of a stock inoculum material for Streptococcus thermophilus strain TH-4

In summary, the production of a stock inoculum material is initiated (step A) by the inoculation of a mother culture (Primary Inoculation Material) containing about 5 x 10⁸ CFU/g into a volume of cultivation medium which is incubated to obtain an inoculum material. The volume of step A is subsequently inoculated into a large volume of cultivation medium which is incubated to obtain a primary fermentation material (step B). Finally, the step B volume is used for inoculation of a still larger volume which is incubated to obtain a fermentation material (step C). The cells are harvested from step C by centrifugation to obtain a concentrate of a starter culture organism cells containing about 10¹¹ CFU per g.

Apparently, the production of a stock inoculum material is substantially the same as the production of a starter culture using conventional methods, i.e. a stepwise propagation. However, as discussed below, a central production of the stock inoculum material is possible such that the inoculum to be used is ready for use as the stepwise propagation of the cells is omitted at the individual starter culture factories.

### Production of the first inoculum material (step A)

The cells present in an ampoule containing 10 grams were used for inoculation of flasks containing 200 ml of 9.5% rehydrated spray-dried skimmed milk powder which was subjected to UHT at 135°C for 8 seconds, followed by an autoclavation at 115°C for 20 min. The inoculum concentration was 1 % weight. The inoculated medium, i.e. the first inoculum material was incubated for 16 hours at the temperature of 37°C.

### Production of the second inoculum material (step B)

A volume of 200 ml of the first inoculum material (obtained in step A) was used for the inoculation of 20 litres of milk 9.5% rehydrated skimmed milk powder to produce the second inoculum material (step B). The inoculation concentration of the first inoculum material was 1 % weight. The second inoculum material was incubated for 9 hours at a temperature of 40°C.

### Production of the final inoculum material (step C)

The volume of the second inoculum material obtained in step B was used for inoculation of 2000 litres of a biomass production medium M17 (OXOID number CM817) to obtain the final inoculum material. The medium was used at the recommended concentration multiplied by 6. The medium was supplemented with 4% lactose solution (w/v). The M17 medium was UHT treated at 145°C for 8 seconds and then cooled to 40°C, and incubated for an appropriate period of time to allow the cells to propagate.

The propagation of the cells was terminated when the consumption of the base had finished by acidification of the lactic acid bacteria producing lactic acid, and the propagated cells were cooled to 10°C.

### 1.2.1.1 Operation parameters during propagation of the cells in step C

The propagation was performed under pH control.

| | |
|---|---|
| Temperature: | 40°C |
| Inoculation concentration: | 1% weight |
| Set point: | pH 6.0 |
| Base: | NH₄OH - 25% |
| Gas in headspace: | N₂ |
| Fermentation time: | 4 hours |

### 1.2.1.2 Production of a concentrate of the cells obtained in step C by centrifugation to obtain a stock inoculum material

After cooling, the propagated cells were subjected to a centrifugation step to obtain a concentrate of the cells. Centrifugation was carried out in a centrifuge resulting in a cell density of 1x10¹¹ cells/gram.

After centrifugation the concentrate was transferred to a sterile container with cooling facilities, and kept at 5°C until freezing. The concentrate was filled directly into sterile non-pyrogenic bags made from a flexible material, and filled into bags each containing 1000 g of the concentrate. Thus, in this production example a stock inoculum material consisting of 100 bags of 1000 g was provided. One of these bags can be used to inoculate 15,000 litres of cultivation medium. After filling, the bags were sealed and frozen in a freezing cabinet with liquid nitrogen sprayed into the cabinet by nozzles for 1 ½ hours, achieving a room temperature of -60°C, and the bags were subsequently stored at -50°C.

### 1.2.2 Production of starter cultures by conventional methods

The so-called commercial DVS (Direct Vat Set) starter cultures, which are highly concentrated cultures for direct inoculation of milk in the dairy industry are chosen as an example of starter cultures produced by conventional methods.

The conventional method of producing such starter cultures begins each time with the stepwise propagation, i.e. in general 2 propagation steps of the cells contained in a mother culture of the cell, in order to be able to produce the necessary amount of inoculum material for the inoculation of the cultivation medium to obtain the starter culture. Normally the inoculation of the cultivation medium is performed with 1% of inoculum material, corresponding to 150 kg per 15,000 litres of cultivation medium.

Subsequently, to complete the propagation, the cultivation medium containing the starter culture cells is concentrated by centrifugation to achieve a concentrate of cells comprising about 1-2 x 10¹¹ cells per gram of cultivation medium. The concentrate may be freeze-dried or frozen in liquid nitrogen as pellets with a diameter of only a few millimetres. The freeze-dried produce is kept at a temperature below 20°C, and the frozen product is kept at a temperature below -50°C until use.

### 1.2.3 Reproducibility of the quality of starter cultures

Tests for product quality such as e.g. metabolic activity and CFU in freeze-dried products produced by the two methods described in 1.2.1 and 1.2.2 were carried out as follows. Product quality, such as e.g. metabolic activity and cell number of the produced starter culture, of 10 different fermentations in milk using starter cultures produced by the two methods, respectively, was evaluated after 4 hours of fermentation by determination of the mean and standard deviation of the fermentations by measuring the cell numbers, metabolic activities and frequency of contamination determined by standard procedures. Metabolic activity is given as the amount of freeze-dried product in grams used to obtain 500 units. 1 unit = the activity of 1 gram of frozen DVS TH-4.

### 1.3 Results of reproducibility

As shown in table 1.1, the variation between the product quality of the 10 fermentations in milk using starter cultures produced by using a subset of each of the 10 fermentations of the same stock inoculum material is small compared to the variation between the quality of starter cultures using the conventional method. Variation is given as means and standard derivations.

**Table 1.1 Means and standard deviations between the metabolic activity and cell number of 10 different fermentations using starter cultures produced by using a subset of a stock inoculum material, and cultures produced in accordance with the conventional method.**

| | Metabolic activity (g/500u) | | Cell number (10¹⁰ cfu/g) | |
|---|---|---|---|---|
| Starter culture | mean | deviation | mean | deviation |
| New method | 91 | 8.6 | 37.9 | 6.35 |
| Conventional | 117.5 | 22.61 | 45.7 | 23.61 |

### 1.4 Conclusion

From the above experiments it can be seen that the fermentation performance of the tested starter cultures was improved and more consistent if the starter cultures were produced by using a subset of the same stock inoculum material, compared to the fermentation performance of starter cultures produced by the conventional method.

Thus, by using the new method it is possible to reduce the variation of the product quality both from batch to batch, but also between factories and plants within a given company. Furthermore, the inoculation system as provided herein permits central preparation of large batches of inoculum material, which can, if required, be stored for extended periods of time, and thereby lead to reduction of workforce. In addition, the method disclosed in the present invention implies a high degree of flexibility as the time for producing a commercial starter culture is reduced considerably.

The stock inoculum material is thus a highly concentrated inoculum material which can be frozen in a transfer enclosure and stored for up to 5 years. Before use, a subset of the stock inoculum material can be thawed, optionally in a water bath for half an hour, and used immediately for inoculation of fermentation material for the preparation of a starter culture.

Thus, there are great advantages of using the new method. First, the conventional method is no longer needed and hereby time, manpower and the cost of raw materials for the stepwise propagation are saved.

Secondly, it is possible to subject the stock inoculum material to various quality tests and procedures before use which ensures a high quality of starter culture when using a subset of the stock inoculation material that possesses high metabolic activity and a high cell number, with substantially no contamination and containing the desired starter culture organisms. For comparisin, when using the conventional method of producing starter cultures, there is often not enough time to subject the inoculum material to all of the below listed tests before the inoculation of the final fermentation medium.
1) Tests for contamination:
   - non-lactic acid bacteria,
   - non-desirable yeast and moulds,
   - coliforms,
   - enterococci,
   - staphylococci,
   - hemolytical bacteria,
   - *Bacillus cereus,*
   - anaerobic gas producing spore formers,
   - lactobacilli and pedicocci;
2) Count of total viable cells, i.e. total number of viable cells per g of culture;
3) Determination of colony morphology in order to secure that the starter culture consists of the desired organisms;
4) Determination of purity, i.e. test for contaminants after 2 times of re-growth in milk;
5) Determination of metabolic activity such as acidification activity by determination of pH reduction in milk after incubation for a specific period at a specific temperature;
6) Phage test in order to determine if the culture contains bacteriophages which may attack the starter culture;
7) API test in order to test which sugar types can be fermented by the starter culture strain. The obtained results are compared with previous results found for this strain (like a fingerprint);
8) Resistance to bacteriophages, i.e. addition of different active bacteriophages to the culture, to which the culture should be resistant;
9) Determination of the content of *Listeria* species and salmonella species;
10) DNA fingerprint and plasmids to ensure that the starter culture comprises the desired organisms;
11) Fermentation tests.

Thirdly, the production planning at the factory producing the starter culture is very flexible, as it only takes a very short time before a new fermenter can be inoculated due to the short time of making the inoculum ready for inoculation, e.g. if the stock is in a frozen state, the thawing of the subset of the stock inoculum material contained in an enclosure takes only about 0.5 to 1 hour for a unit of 1000 g.

In summary, it can be said that one batch, i.e. one production of a stock inoculum material results in a high number of enclosures which consist of the same inoculum material, and allow a production of starter cultures with a consistent quality.

### EXAMPLE 2

### Production of a stock inoculum material of yeast

This example describes the production of a stock inoculum material of *Debaryomyces hansenii* strain LAF-3 which is useful in the present invention.

### 2.1 Material and methods

In summary, the production of a stock is initiated (step A) by the inoculation of a mother culture (Primary Inoculation Material) containing 1 x 10⁸ CFU/g into a volume of a cultivation medium which is incubated aerobically to obtain an inoculum material. The volume of step A is subsequently inoculated into a large volume of cultivation medium which is incubated aerobically to obtain the final fermentation material (step B). The yeast cells are harvested from step B by centrifugation to obtain a concentrate of starter culture organism cells containing about 5 x 10⁸ CFU per gram.

### 2.1.1 Production of the first inoculum material (step A)

The yeast cells, contained in an ampoule of 10 grams, were used for inoculation in a 3 litres fermenter containing 2800 ml of the medium as shown in table 2.1.

**Table 2.1 Content of the medium used for the production of the first inoculum material**

| **Component** | **Origin** | **g/L** |
|---|---|---|
| Yeast Extract | Oxoid L21 | 20.0 |
| MgSO₄.7H₂O | Merck 105882 | 10.0 |
| Dextrose | Merck 108337 | 10.0 |
| Antifoam | | 0.3 |
| Water | Tap water | 959.7 |

The medium was sterilised by an autoclavation at 121°C for 20 min. The dextrose was autoclaved separately.

The propagation of the yeast was performed under pH control:

| | |
|---|---|
| Temperature | 25°C |
| pH | pH 5.8 is adjusted in the medium prior to inoculation |
| pH setpoint | pH 5.5-5.8 (adjusted by 25% w/w NH₄OH) |
| aeration | 2.5 I/min |
| fermentation time | 24 hours |

### 2.1.2 Production of the final inoculum material (step B)

The volume of the first inoculum material (obtained in step A) was used for inoculation in a 750 litres Chemap fermenter containing 400 litres of medium. The medium composition is as shown in table 2.2.

**Table 2.2 Content of the medium used for the production of the final inoculum material**

| **Component** | **Origin** | **g/L** |
|---|---|---|
| Yeast Extract | Oxoid L 21 | 20.0 |
| MgSO₄.7H₂O | Merck 105882 | 10.0 |
| Dextrose | Merck 108337 | 10.0 |
| ZnSO₄.7H₂O | Merck 108881 | 1.0 |
| Vitamin solution | (*) | 0.1 |
| Antifoam | | 0.3 |
| Water | Tap water | 959.6 |
| * Vitamin solution | | |

| **Component** | **Origin** | **g/L** |
|---|---|---|
| Riboflavin | Merck 500257 | 13.0 |
| Thiaminmononitrate | Merck 500980 | 13.0 |
| Pyridoxalhydrochloride | Merck 500224 | 13.0 |
| Calcium D-pantothenate | Merck 440744Y | 13.0 |
| D(+)-Biothin | Merck 500030 | 1.3 |
| Nicotinic acid | Merck 481918 | 40.0 |
| Water | (distilled water) | 1000.0 |
| sterilisation temperature | UHT treatment of the medium at 144°C for 8 sec. 25°C | |
| pH | pH 5.8 is adjusted in the medium prior to inoculation | |
| pH setpoint aeration | pH 5.5-5.8 (adjusted by 25% w/w NH₄OH) 370 l/min | |
| | | |
| fed batch | glucose added per hour after incubation for T hours. | |
| feed profile | T=0 | 12 kg 33.3% glucose |
| | T=17.5 | 1 kg/hour (33.3% glucose) |
| | T=21 | 3 kg/hour (33.3% glucose) |
| | T=23.75 | dosing was stopped |
| fermentation time | 24 hours | |

### 2.1.3 Production of a concentrate of the yeast cells obtained in step B by centrifugation to obtain a stock of inoculum material

After cooling, the propagated cells were subjected to a centrifugation step to obtain a concentrate of the cells. Centrifugation was carried out in a centrifuge resulting in a cell density of 5x10⁸ cells/gram. 20 % of glycerol were added to the concentrate.

After centrifugation the concentrate was transferred to a sterile container with cooling facilities, and kept at 5°C until freezing. The concentrate was filled directly into sterile non-pyrogenic bags made from a flexible material, each containing 1000 g of the concentrate. Thus, in this production example a stock inoculum material consisting of 100 bags of 1000 g each was provided. One of these bags can be used to inoculate 5,000 litres of cultivation medium. After filling, the bags were sealed and frozen in a freezing cabinet with liquid nitrogen sprayed into the cabinet by nozzles for 1½ hours, achieving a room temperature of -60°C. Subsequently the bags were stored at -50°C.

### 2.2 Result and conclusion

From the above experiment it can be seen that it is possible to provide a stock of yeast inoculum comprising a concentrate of starter culture organism cells of about 5 x 10⁸ CFU per gram. This stock of viable yeast cells can be used for direct inoculation of a suitable cultivation medium, in order to produce a starter culture useful for the fermentation industry.

### EXAMPLE 3

### Production of a stock inoculum material of various useful starter culture organisms

In this example it is shown that it is possible to produce a stock inoculum material of different kinds of organisms, such as lactic acid bacteria and Gram-negative bacteria. The following microbial species are used in this example:
*Bacillus licheniformis* (CH 200) and *Bacillus subtilis* (CH 201) which are used for the commercial product Bioplus, a biological growth promoter in e.g. cattle fodder;
*Bacillus cereus* strain BP-01 is a non-toxical bacterial strain which is used as a soil treatment/improvement, in connection with cotton plants;
*Enterococcus faecium* strain SF 202, 273 & 301 is used for the production of silage;
*Lactococcus lactis sp. lactis* strain BMK16L, is used for the production of Nisin, which is an additive used in processed cheese;
*Pseudomonas chlororaphis* strain MA 342, is a Gram-negative rod that is used as a biological seed treatment product that prevents and controls plant diseases.

### Production procedure

The production of a stock of each of the above organisms is essentially the same as described in example 1 and 2, and is thus initiated by the inoculation of a mother culture (Primary Inoculation Material) containing 5 x 10⁸ CFU/g into a volume of a cultivation medium which is incubated aerobically to obtain an inoculum material. The volume of step A is subsequently inoculated into a large volume of cultivation medium which is incubated aerobically to obtain the final fermentation material (step B). The cells are harvested from step B by centrifugation (step C) to obtain a concentrate of starter culture organism cells containing about 5 x 10⁸ - 2 x 10¹¹ CFU per gram.

### Fermentation conditions

### Media used for the fermentation:

*Bacillus* and *Pseudomonas* species are fermented in TSB (Tryptic Soy Broth, Difco 0370-*17). Enterococcus* species are fermented in M.R.S Broth (Oxoid, CM359) and *Lactococcus lactis sp. lactis* is fermented in KK-97-6 (Chr. Hansen medium)

### Fermentation parameters

For all *Bacillus* species the incubation temperature is 37°C, pH is maintained at 7.0 during all steps of the fermentation, the fermentation time in step A is about 24 hours, and in step B the fermentation time is about 20 hours, and in step C the time is about 30 hours. For all *Enterococcus* species the incubation temperature is 30°C, pH is maintained at 5.6 during all steps of the fermentation, the fermentation time in step A is about 24 hours, in step B about 12 hours, and in step C about 20 hours. For the *Pseudomonas* species the incubation temperature is 28°C, pH is not maintained during the fermentation.

All species are separated at the highest possible concentration degree and subsequently filled into enclosures, making up a stock of inoculum material useful for the production of a specific starter culture, used in the method according to the invention.

## Claims

1. A method for supply of a starter culture with a consistent quality comprising the steps of:
(i) supply of a stock inoculum material comprising a concentrate of starter culture organism cells;
(ii) use of, for subsequent production of starter cultures, a subset of said stock inoculum material for direct inoculatiion of a cultivation medium with said starter culture organism;
(iii) propagation of the cells of the starter culture organism for a period of time adjusted sufficiently in size to produce a desired amount of said cells; and
(iv) harvest of the propagated cells to provide a starter culture.

2. A method according to claim 1, wherein the stock inoculum material provided in step (i) is in quantities sufficient to inoculate at least 50,000 litres of cultivation medium.

3. A method according to claim 1, wherein the concentrate provided in step (i) contains at least 10⁸ CFU per g.

4. A method according to claim 1, wherein the subset of the stock inoculum material in step (ii) is directly inoculated in the cultivation medium at a rate of maximum 0.1%.

5. A method according to claim 1, wherein the amount of the subset of the stock inoculum material for direct inoculation of the cultivation medium in step (ii) provides a ratio of the CFU per g of cultivation medium, immediately after inoculation, relative to the CFU per g of the subset of the stock inoculum material to be inoculated, said ratio being in the range from 1:100 to 1:100,000.

6. A method according to claim 1, wherein the cultivation medium immediately after the inoculation in step (ii) contains a number of CFU per g of cultivation medium which is at least 10⁵.

7. A method according to any of the claims of 1 to 6, wherein the stock inoculum material is provided in sealed enclosures.

8. A method according to claim 7, wherein the sealed enclosures have a cubic content of at least 0.01 litre.

9. A method according to claim 7, wherein the sealed enclosures are provided with outlet means for connection of the enclosure to the container comprising the liquid cultivation medium, said outlet means permitting the concentrate of cells to be introduced substantically aseptically into the container to inoculate the liquid cultivation medium with said concentrate.

10. A method according to any of the claims of 1 to 9, wherein the starter culture organism in step (i) originates from a species selected from the group consisting of a lactic acid bacterial species, a *Bifidobacterium* species, a *Propionibacterium* species, a *Staphylococ*cus species, a *Micrococcus* species, a *Bacillus* species, an *Enterobacteriaceae* species including *E. coli,* an *Actinomycetes* species, a *Corynebacterium* species, a *Brevibacterium* species, a *Pediococcus* species, a *Pseudomonas* species, a *Sphingomonas* species, a *Mycobacterium* species, a *Rhodococcus* species, a fungal species and a yeast species.

11. A method according to claim 10, wherein the lactic acid bacterial species is selected from the group consisting of *Lactococcus* spp., *Lactobacillus* spp., *Leuconostoc* spp., Pe*diococcus* spp., *Oenococcus* spp. and *Streptococcus* spp.

12. A method according to any of the claims of 1 to 11, wherein the stock inoculum material in step (i) comprises at least two starter culture strains.

13. A method according to any of the claims of 1 to 12, wherein the starter culture is used for inoculation of milk which is further processed to obtain a dairy product, which is selected from the group consisting of cheese, yoghurt, butter, inoculated sweet milk and a liquid fermented milk product.

14. A method according to any of the claims of 1 to 13, wherein the cells being propagated in the cultivation medium express a desired gene product or produce a desired product.

15. A method according to claim 14, wherein the desired gene product is selected from the group consisting of enzymes, pharmaceutically active substances, polysaccharides and amino acids.
